# EUROPÄISCHE PATENTSCHRIFT

(11) **EP 3 810 053 B1**
(45) Veröffentlichungstag und Bekanntmachung des Hinweises auf die Patenterteilung: **18.06.2025**
(21) Anmeldenummer: 19732619.2
(22) Anmeldetag: 19.06.2019
(51) Int. Cl.: A61F 13/38, A61J 1/00, B65D 71/00, B65D 81/00

(54) **ZUFÜHRVORRICHTUNG UND BEHÄLTER DAFÜR SOWIE BESCHICKUNGSVERFAHREN**
FEEDING DEVICE, CONTAINER THEREFOR, AND SUPPLYING METHOD
DISPOSITIF D'ALIMENTATION, RÉCIPIENT ASSOCIÉ, ET PROCÉDÉ D'ALIMENTATION

(30) Priorität: 19.06.2018 DE 202018002854 U
(43) Veröffentlichungstag der Anmeldung: 28.04.2021
(73) Patentinhaber: Setter Holding GmbH, 46446 Emmerich (DE)
(72) Erfinder: GASSE, Steffen, 54534 Großlittgen (DE); HÜLKENBERG, Roland, 46446 Emmerich am Rhein (DE); HERBERG, Pascal, 47533 Kleve (DE); MENNEMANN, Marcel, 47533 Kleve (DE); KNIST, Florian, 46446 Emmerich am Rhein (DE)
(74) Vertreter: Grosse Schumacher Knauer von Hirschhausen
(86) Internationale Anmeldenummer: PCT/EP2019/066268
(87) Internationale Veröffentlichungsnummer: WO 2019/243451

(56) Entgegenhaltungen:
- WO-A1-2012/139231
- CN-B- 103 006 386
- CN-U- 204 734 850
- US-A- 3 263 280
- US-A- 3 389 436

## Beschreibung

### GEBIET DER ERFINDUNG

Die Erfindung betrifft ein Verfahren zum Beschicken nach dem Oberbegriff des Anspruchs 1. Die in dem Verfahren verwendete Zuführeinrichtung dient demnach dem Zuführen einer einem Großgebinde entsprechenden Anzahl von länglichen, insbesondere stabförmigen, Papierkörpern in zumindest eine Verarbeitungseinrichtung, in der jeder längliche, insbesondere stabförmige, Papierkörper ausschließlich dann verarbeitbar ist, wenn er sich in einer definierten Verarbeitungslage befindet. Die erforderliche Verarbeitungslage umfasst zumindest Position und Orientierung des zu verarbeitenden Papierkörpers. In einer von der Verarbeitungslage abweichenden Lage/Position/Orientierung ist eine Verarbeitung des Papierkörpers in der Verarbeitungseinrichtung nicht oder nur fehlerhaft möglich. Ein Großgebinde umfasst mehrere Tausend, mehrere Zehntausend oder mehrere Hunderttausend Papierkörper, je nach Papierkörperabmessung respektive Papierkörpergestalt.

Die länglichen, insbesondere stabförmigen, Papp- oder Papierkörper können beispielsweise als Papierstäbchen oder -röhrchen oder -sticks, oder als Papier- oder Pappplättchen gebildet sein. Derartige Papierkörper werden in großen Stückzahlen (Großgebinde) von einer Fertigungseinrichtung gefertigt und bedürfen nach der Fertigung regelmäßig einer maschinellen Nach- oder Weiterverarbeitung. Bei Wattestäbchen beispielsweise besteht eine Verarbeitungsmaßnahme darin, endseitig Watte an dem länglichen Papierkörper (Stick) anzubringen. Bei Spateln oder Plättchen beispielsweise ist eine sich an die Fertigung anschließende Walz- oder Umformverarbeitung zweckmäßig. Eine weitere Verarbeitungsmaßnahme kann ein Bedrucken, Beschriften oder Kennzeichnen der gefertigten Papierkörper betreffen. Derartige Verarbeitungsmaßnahmen werden in geeigneten apparativen Verarbeitungseinrichtungen umgesetzt. Mit der Zuführvorrichtung werden die gefertigten Papierkörper einer Verarbeitungseinrichtung oder mehreren Verarbeitungseinrichtungen apparativ zugeführt.

Weiter beschrieben wird ein wiederverwendbarer Behälter. Demnach dient der Behälter dem Transportieren von länglichen, insbesondere stabförmigen, Papierkörpern einer einem Großgebinde entsprechenden Anzahl von Papierkörpern von einer ersten Einrichtung, insbesondere einer Fertigungseinrichtung, in zweite, als Zuführvorrichtung gebildete Einrichtung. Auch eine Verwendung eines wiederverwendbaren Behälters wird beschrieben.

Die Erfindung betrifft ein Verfahren zum Beschicken zumindest einer Verarbeitungseinrichtung zum Verarbeiten von länglichen, insbesondere stabförmigen, Papierkörpern einer einem Großgebinde entsprechenden Anzahl von Papierkörpern nach Anspruch 1.

Schließlich wird auch ein Verteilsystem beschrieben.

Demnach dient das Verteilsystem zum Verteilen von länglichen, insbesondere stabförmigen, Papierkörpern einer einem Großgebinde entsprechenden Anzahl von Papierkörpern auf mehrere Verarbeitungseirichtungen, worin der Papierkörper ausschließlich dann verarbeitbar ist, wenn er sich in einer definierten Verarbeitungslage (Position/Orientierung) befindet.

### TECHNOLOGISCHER HINTERGRUND

Nach der Fertigung der länglichen, insbesondere stabförmigen, Papierkörper werden diese in Großgebinde, beispielsweise Kartons mit etwa einigen 1.000, etwa einigen 10.000 oder etwa einigen 100.000 Stück Papierkörpern, verpackt und die verpackten Papierkörper werden in den Verpackungskartons zu einer Verarbeitungseinrichtung transportiert. Da bei dem Verarbeiten eine definierte Verarbeitungsposition oder -lage der Papierkörper erforderlich ist, erfolgt das Verpacken der länglichen Papierkörper geordnet respektive gerichtet, d. h. die Papierkörper werden in einer bestimmten, definierten Richtung in die Verpackung gelegt. Dies ist aufwändig und fehleranfällig, so dass es immer wieder vorkommt, dass einzelne Papierkörper schief und außerhalb der gewünschten Ordnung in die Verpackung gelangen.

Zum (Weiter-)Verarbeiten der Papierkörper werden die befüllten Großgebinde-Verpackungskartons mit den (ordentlich) verpackten länglichen Papierkörpern zunächst geöffnet und sodann in eine Verarbeitungseinrichtung gegeben, wo ein geordnetes Entnehmen der länglichen Papierkörper aus den Verpackungskartons erfolgen soll.

US3389436 betrifft eine Verarbeitungsmaschine für Papierstäbe.

Abweichungen von der Sortierung und/oder Ordnung der Papierkörper innerhalb der Verpackung führen regelmäßig zu Verzögerung beim Verarbeiten der länglichen Papierkörper. Es bedarf beispielsweise einer manuellen Korrektur, wenn ein Papierkörper schief in der Verpackung liegt und dann in der Verarbeitungseirichtung nicht oder nur fehlerhaft bearbeitbar ist, da der Papierkörper nicht die erforderliche Verarbeitungsposition respektive Verarbeitungslage hat. Erst nach einem manuellen Wiederherstellen der definierten Verarbeitungslage des schief oder abweichend liegenden Papierkörpers innerhalb der Verarbeitungseinrichtung kann die Verarbeitung fortgesetzt werden. Hier setzt die Erfindung ein.

### DARSTELLUNG DER ERFINDUNG

Die zu lösende Aufgabe besteht insofern darin, die Handhabung länglicher, insbesondere stabförmiger, Papierkörper zu vereinfachen, insbesondere im Hinblick auf ein verbessertes Zuführen der Papierkörper in Verarbeitungseinrichtungen. Diese Aufgabe wird durch Verfahren zum Bestücken nach Anspruch 1 gelöst. Die in dem Verfahren verwendete Zuführvorrichtung umfasst zumindest eine Ausrichteinrichtung, mit welcher einzelne oder sämtliche länglichen, insbesondere stabförmigen, Papierkörper der einem Großgebinde entsprechenden Anzahl von Papierkörpern so ausrichtbar sind, dass sich die länglichen, insbesondere stabförmigen, Papierkörper bereits in der definierten Verarbeitungslage befinden, wenn sie zu der oder in die Verarbeitungseinrichtung gelangen. Ein manuelles Ausrichten entfällt. Eine Vorsortierung respektive ein Orientieren der länglichen Papierkörper vor dem Zuführen der Papierkörper in die Verarbeitungseinrichtung entfallen ebenfalls.

Bevorzugt können die länglichen, insbesondere stabförmigen, Papierkörper über eine Fördereinrichtung, insbesondere über ein Förderband, in die Ausrichteinrichtung der Zuführeinrichtung gelangen. Der Transport der länglichen Papierkörper auf dem Förderband kann ungerichtet, d. h. ohne bevorzugte Orientierung der Papierkörper, erfolgen, da in der hinter der Fördereinrichtung angeordneten Ausrichteirichtung das Ausrichten respektive Orientieren der länglichen Papierkörper erfolgt.

Zumindest ein wiederverwendbarer Behälter ist vorgesehen mit dem die länglichen, insbesondere stabförmigen, Papierkörper in die Zuführvorrichtung, insbesondere als Schüttgut, gegeben werden, entfällt der Bedarf an Verpackungskartons für die länglichen Papierkörper auf dem Weg in die Verarbeitungseinrichtung vollständig. Es müssen demnach weder Kartons beschafft und bereitgehalten werden, noch müssen die entleerten Kartons zerlegt und entsorgt werden. Dies reduziert Kosten und eine Belastung der Umwelt. Bevorzugt kann der wiederverwendbare Behälter stapelbar sein.

Sofern der wiederverwendbare Behälter so groß ist, dass mehrere Zehntausend oder mehrere Hunderttausend längliche Papierkörper darin Platz finden, ist eine Entleerungsstation zweckmäßig, worin der wiederverwendbare Behälter so entleert wird, dass die länglichen, insbesondere stabförmigen, Papierkörper in die Zuführvorrichtung gelangen. Das Entleeren kann mit zumindest einer, insbesondere hydraulischen, Hub- und/oder Kippeinrichtung erfolgen, welche Bestandteil der Entleerungsstation ist.

Die Papierkörper können die Form eines Stabes oder Sticks haben, also im Wesentlichen zylindrisch mit im Wesentlichen rundem Querschnitt. Die Papierkörper können hohl sein, beispielsweise in Gestalt eines Hohlzylinders. Die Papierkörper können als längliche, flache Platten oder Plättchen gebildet sein, etwa mit im Wesentlichen rechteckigem oder ovalem Querschnitt.

Eine Vereinfachung der Handhabung länglicher, insbesondere stabförmiger, Papierkörper, insbesondere im Hinblick auf ein verbessertes Zuführen der Papierkörper in Verarbeitungseinrichtungen dafür, ergibt sich außerdem durch einen wiederverwendbaren Behälter**.**

Der Behälter dient dem Transportieren von länglichen, insbesondere stabförmigen, Papierkörpern von einer ersten Einrichtung, insbesondere einer Fertigungseinrichtung, in zumindest eine zweite, als Zuführvorrichtung gebildete Einrichtung, insbesondere in eine hierin beschriebene Zuführvorrichtung. Der wiederverwendbare Behälter eignet sich zum Transportieren von bis zu mehreren Tausend, Zehntausend oder Hunderttausend Papierkörpern, d. h. für den Transport von entsprechenden Großgebinden.

Eine Vereinfachung der Handhabung länglicher, insbesondere stabförmiger, Papierkörper, insbesondere im Hinblick auf ein verbessertes Zuführen der Papierkörper in Verarbeitungseinrichtungen dafür, ergibt sich ferner durch einen Verwendung eines, insbesondere hierin beschriebenen, wiederverwendbaren Behälters.

Der wiederverwendbare Behälter macht Einwegverpackungen, insbesondere Pappkartons, zum Transportieren der länglichen, insbesondere stabförmigen, Papierkörper entbehrlich.

Eine Vereinfachung der Handhabung länglicher, insbesondere stabförmiger, Papierkörper, insbesondere im Hinblick auf ein verbessertes Zuführen der Papierkörper in Verarbeitungseinrichtungen dafür, ergibt sich außerdem durch ein Verfahren zum Beschicken zumindest einer Verarbeitungseinrichtung zum Verarbeiten von länglichen, insbesondere stabförmigen, Papierkörpern einer einem Großgebinde entsprechenden Anzahl von Papierkörpern, nach Anspruch 1.

Bei dem Verfahren werden die länglichen, insbesondere stabförmigen, Papierkörper mittels zumindest einem, insbesondere hierin beschriebenen, wiederverwendbaren Behälter, zumindest einer, insbesondere hierin beschriebenen, Zuführvorrichtung zugeführt. Die länglichen, insbesondere stabförmigen, Papierkörper werden in der Zuführvorrichtung so ausgerichtet, dass die länglichen, insbesondere stabförmigen, Papierkörper in eine definierte Verarbeitungslage gebracht werden, so dass die länglichen, insbesondere stabförmigen, Papierkörper in der definierten Verarbeitungslage zumindest einer Verarbeitungseinrichtung zuführbar sind.

Es kann vorgesehen sein, dass die länglichen, insbesondere stabförmigen, Papierkörper in zumindest einer Entleerungsstation aus dem wiederverwendbaren Behälter in die Zuführvorrichtung gelangen. Die länglichen, insbesondere stabförmigen, Papierkörper können über zumindest eine Fördereinrichtung zu zumindest einer die länglichen, insbesondere stabförmigen, Papierkörper ausrichtenden Ausrichteinrichtung gefördert werden.

Eine Vereinfachung der Handhabung länglicher, insbesondere stabförmiger, Papierkörper, insbesondere im Hinblick auf ein verbessertes Zuführen der Papierkörper in Verarbeitungseinrichtungen dafür, ergibt sich außerdem durch ein Verteilsystem zum Verteilen von länglichen, insbesondere stabförmigen, Papierkörpern auf mehrere Verarbeitungseirichtungen.

Das Verteilsystem umfasst eine hierin beschriebene Zuführvorrichtung, wobei in dem System ein hierin beschriebenes Beschickungsverfahren abläuft.

Die vorgenannten sowie die beanspruchten und in den Ausführungsbeispielen beschriebenen erfindungsgemäß zu verwendenden Bauteile unterliegen in ihrer Größe, Formgestaltung, Materialauswahl und technischen Konzeption keinen besonderen Ausnahmebedingungen, so dass die in dem Anwendungsgebiet bekannten Auswahlkriterien uneingeschränkt Anwendung finden können.

Weitere Einzelheiten, Merkmale und Vorteile des Gegenstandes der Erfindung ergeben sich aus den Unteransprüchen, sowie aus der nachfolgenden Beschreibung und der zugehörigen Zeichnung, in der - beispielhaft - ein Ausführungsbeispiel eines Verteilsystems mit einer Zuführvorrichtung dargestellt ist. Auch einzelne Merkmale der Ansprüche oder der Ausführungsformen können mit anderen Merkmalen anderer Ansprüche und Ausführungsformen kombiniert werden.

### KURZBESCHREIBUNG DER FIGUREN

In der Zeichnung zeigen
- Fig. 1: ein Schema eines Verteilsystems gemäß dem Stand der Technik und
- Fig. 2: eine Schema eines Verteilsystems mit erfindungsgemäßer Zuführvorrichtung.

### DETAILLIERTE BESCHREIBUNG VON AUSFÜHRUNGSBEISPIELEN

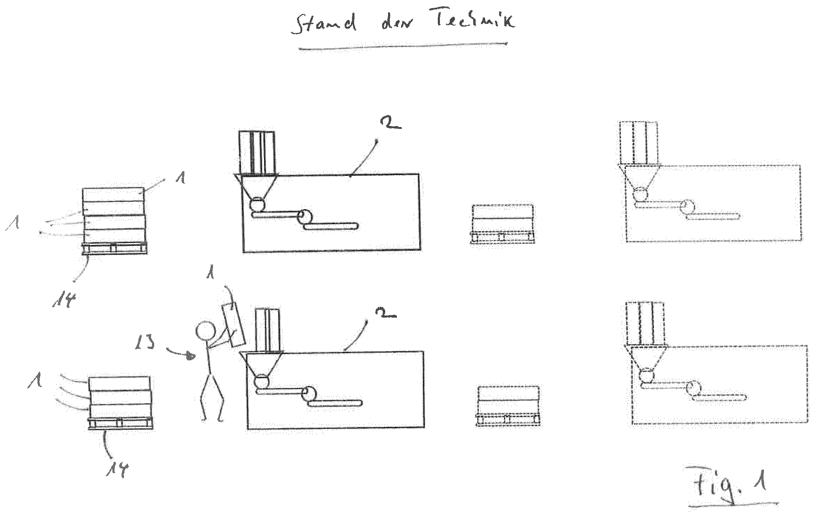

Bei der industriellen Fertigung von Papierstäbchen, etwa für Wattestäbchen, werden nicht selten Stückzahlen von mehreren Zehntausend pro Charge gefertigt und anschließend einer Weiterverarbeitung zugeführt. Dabei ist es Stand der Technik (Fig. 1), die gefertigten Sticks in (Papp-)Kartons 1 zu verpacken. Die Kartons 1 werden, wie in Fig. 1 schematisch dargestellt, zu einer Verarbeitungseinrichtung 2 getragen, dort geöffnet und - etwa im 10-Minuten-Takt - in die Verarbeitungseinrichtung 2 eingesetzt. In ähnlicher Weise wird verfahren, wenn nicht Sticks sondern flache oder hohle Papp- oder Papierkörper gefertigt und weiterverarbeitet werden sollen.

Das Tragen und Einsetzten der Verpackungskartons 1 ist mühsam, insbesondere wenn in einem Karton 1 beispielsweise mehrere Zehntausend gefertigter Papierkörper (Stäbchen) enthalten sind. Regelmäßig muss dabei aufgrund der Maschinendimensionen über Kopfhöhe gearbeitet werden.

Die gefertigten Stäbchen respektive Papierkörper werden nach der Fertigung sortiert und orientiert in die Kartons 1 gefüllt, damit die Stäbchen/Körper in einer definierten Verarbeitungslage der Verarbeitungseinrichtung 2 zugeführt werden können. Ein schief verpacktes Stäbchen, also ein Stäbchen, welches sich nicht in der definierten Verarbeitungslage befindet, wird in der Verarbeitungseinrichtung 2 als Störung identifiziert oder verursacht dort eine Störung und es bedarf dann jeweils eines manuellen Eingriffs, etwa durch einen Techniker 13. Eine Beseitigung der Störung durch das "außerordentliche" Stäbchen erfordert stets Übung und Geduld des eingreifenden Technikers 13. Bei dem manuellen Eingriff wird die Position respektive Lage des schief sitzenden Stäbchens mitunter so korrigiert, dass das Stäbchen anschließend wieder die gewünschte und definierte Verarbeitungslage einnimmt. Diese Korrektur verlangsamt die Verarbeitung innerhalb der Verarbeitungseinrichtung 2 und macht den gesamten Fertigungs- und VerarbeitungsProzess kostenintensiv.

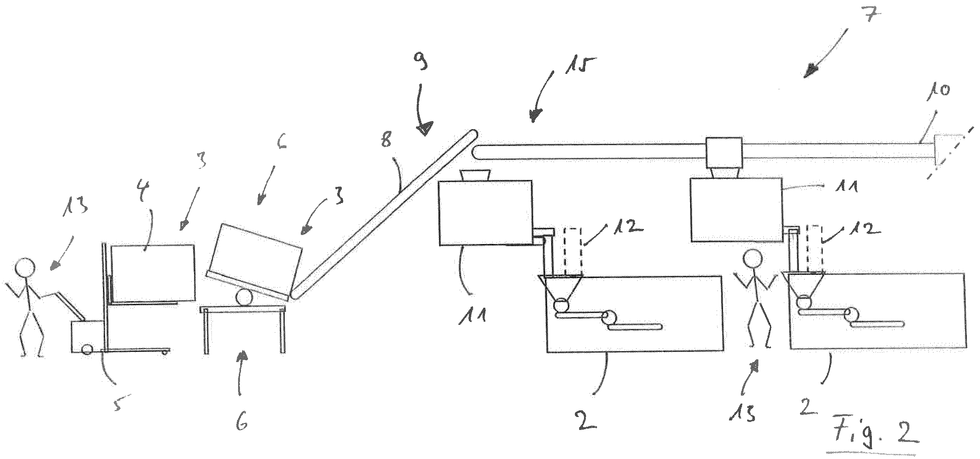

Gemäß der Erfindung ist ein Vorsortieren und Ordnen der gefertigten Stäbchen/Papierkörper nicht mehr erforderlich. Die Stäbchen werden, wie in Fig. 2 schematisch gezeigt, nach der Fertigung als Schüttgut 3 in einen wiederverwendbaren Behälter 4 gegeben, der, etwa mit einem Hubwagen oder Stapler 5, zu einer Entleerungsstation 6 eines Verteilsystems 7 mit Zuführvorrichtung 15 gebracht wird. Dort wird der wiederverwendbare Behälter 4 entleert und die unsortierten und nicht orientierten Stäbchen werden auf ein Förderband 8 einer Fördereinrichtung 9 gegeben. Von dort gelangen die Stäbchen/Papierkörper zu zumindest einer Verarbeitungseinrichtung 2, wobei gemäß Fig. 2 zwei Verarbeitungseinrichtungen 2 schematisch abgebildet sind. Es können weitere Verarbeitungseinrichtungen 2 in dem Verteilsystem 7 vorgesehen sein, wie die in Fig. 2 strichliert dargestellte Förderstrecke 10 symbolisieren soll.

Bezogen auf die Förderrichtung ist hinter der Entleerungsstation 6 und vor der Verarbeitungseinrichtung 2 ein Ausricht-Einrichtung 11 vorgesehen, in der die geförderten Papierkörper ausgerichtet werden, so dass die Papierkörper in der definierten Verarbeitungs-Lage in die Verarbeitungseinrichtung 2 gelangen.

Im Hinblick auf eine Notversorgung 12 der Verarbeitungseinrichtungen 2 kann eine Aufnahme für herkömmliche Verpackungskartons 1 vorgesehen sein, etwa falls die Entleerungsstation 6 oder die Fördereinrichtung 9 technische Störungen haben sollten.

Mit dem in Fig. 2 gargestellten Verteilsystem 7 wird erreicht, dass gefertigte Stäbchen nicht mehr aufwändig vorsortiert und verpackt werden müssen, damit diese weiterverarbeitbar sind. Das personalaufwändige Verpacken in Kartons 1 entfällt. Es entfällt auch eine personalaufwändige Kontrolle beim Verpackungsvorgang. Somit müssen Kartons 1 weder bestellt und bereitgehalten werden, noch müssen die Kartons 1 nach Beschickung der Verarbeitungseinrichtungen 2 entsorgt werden. Durch den Entfall der Kartonverpackung 1 wird die Fertigung und (Weiter-) Verarbeitung in ökologischer Hinsicht nachhaltiger. Es ist auch nicht mehr erforderlich, die mitunter schweren Kartons 1 (über Kopf) in die Verarbeitungseinrichtung(en) 2 einzusetzen und dort zu justieren. Ein manueller Eingriff bei schief verpackten Stäbchen/Papierkörpern entfällt ebenfalls, da das Verteilsystem 7 mit der Ausrichteinrichtung 11 jedes Stäbchen in die definierte Verarbeitungslage ausrichtet. Die Notwendigkeit manueller Korrekturen und sich ständig wiederholender Bedienungen durch einen oder mehrere routinierte Techniker 13 wird erheblich reduziert, was den gesamten Fertigungs- und Verarbeitungsprozess beschleunigt und effizienter werden lässt, auch im Hinblick auf die Kosten.

Die Papierkörperfertigung und die Papierkörper-Verarbeitung können an verschiedenen Orten stattfinden. In diesem Fall werden die wiederverwendbaren Behälter 4 auf Paletten 14 gestapelt und vom Ort der Fertigung zum Ort der (Weiter-) Verarbeitung etwa mittels Spedition transportiert. Am Zielort, d. h. am Ort der Verarbeitung, werden die wiederverwendbaren Behälter 4 geöffnet und die darin unsortiert enthaltenen Papierkörper werden in das in Fig. 2 schematisch dargestellte Verteilsystem 7 geschüttet. Bei einem derartigen Prozessablauf hält der Vorrat an Papierkörpern für mehrere Stunden, so dass die Verarbeitungseinrichtung(en) 2 spürbar geringere (Still-)Standzeiten erreichen. Schließlich gestatten die Verwendung des gezeigten Verteilsystems 7 respektive die Durchführung der darin ablaufenden Beschickungsverfahren, dass eine Papierkörperversorgung mehrerer Fabriken, welche jeweils Papierkörper-Verarbeitungseinrichtungen betreiben, etwa an mehreren Standorten, zentral organisiert werden kann.

### BEZUGSZEICHENLISTE

- 1: (Papp-)Karton
- 2: Verarbeitungseinrichtung
- 3: Schüttgut
- 4: Behälter
- 5: Stapler
- 6: Entleerungseinrichtung
- 7: Verteilsystem
- 8: Förderband
- 9: Fördereinrichtung
- 10: Förderstrecke
- 11: Ausrichteinrichtung
- 12: Notversorgung
- 13: Techniker
- 14: Palette
- 15: Zuführvorrichtung

## Patentansprüche

1. Verfahren zum Beschicken zumindest einer Verarbeitungseinrichtung (2) zum Verarbeiten von länglichen, stabförmigen Papierkörpern einer einem Großgebinde entsprechenden Anzahl von Papierkörpern, wobei die Anzahl mindestens mehrere tausend Papierkörper umfasst, wobei bei dem Verfahren die länglichen, stabförmigen Papierkörper mittels zumindest einem wiederverwendbaren Behälter (4), in dem die länglichen, stabförmigen Papierkörper als unsortiertes und nicht orientiertes Schüttgut angeordnet sind, zumindest einer Zuführvorrichtung (15) der Verarbeitungseinrichtung (2) zugeführt werden, wobei die länglichen, stabförmigen Papierkörper der einem Großgebinde entsprechenden Anzahl von Papierkörpern mittels zumindest einer Entleerungsstation über zumindest eine Fördereinrichtung (9) in einer Ausrichteinrichtung (11) der Verarbeitungseinrichtung (2) so ausgerichtet werden, dass die länglichen, stabförmigen Papierkörper in eine definierte, Position und Orientierung jedes länglichen, stabförmigen Papierkörpers umfassende Verarbeitungslage gebracht werden, so dass die länglichen, stabförmigen Papierkörper in der definierten Verarbeitungslage der Verarbeitungseinrichtung zuführbar sind, in der jeder längliche, stabförmige Papierkörper ausschließlich dann verarbeitbar ist, wenn er sich in der definierten Verarbeitungslage befindet, und wobei einzelne oder sämtliche länglichen, stabförmigen Papierkörper der einem Großgebinde entsprechenden Anzahl von Papierkörpern in der Ausrichteinrichtung (11) so ausgerichtet werden, dass sich die länglichen, stabförmigen Papierkörper bereits in der definierten Verarbeitungslage befinden, wenn sie zu der oder in die Verarbeitungseinrichtung (2) gelangen.

## Claims

1. A method for supplying at least one processing apparatus (2) for the processing of elongate, rod-shaped paper bodies of a number of paper bodies corresponding to a large receptacle, wherein the number comprises at least several thousand paper bodies, wherein in the method the elongate, rod-shaped paper bodies are fed by means of at least one reusable container (4), in which the elongate, rod-shaped paper bodies are arranged as unsorted and non-oriented bulk material, to at least one feeding device (15) of the processing apparatus (2), wherein the elongate, rod-shaped paper bodies of the number of paper bodies corresponding to a large receptacle are oriented by means of at least one emptying station via at least one conveying apparatus (9) in an orienting apparatus (11) of the processing apparatus (2) so that the elongate, rod-shaped paper bodies are brought into a defined processing position comprising position and orientation of each elongate, rod-shaped paper body, so that the elongate, rod-shaped paper bodies are able to be fed in the defined processing position to the processing apparatus, in which each elongate, rod-shaped paper body can be processed only if it is situated in the defined processing position, and wherein individual or all elongate, rod-shaped paper bodies of the number of paper bodies corresponding to a large receptacle are oriented in the orienting apparatus (11) so that the elongate, rod-shaped paper bodies are already in the defined processing position when they reach or enter the processing apparatus (2).

## Revendications

1. Procédé, destiné à charger au moins une installation de traitement (2), destinée à traiter des corps en papier allongés, en forme de barres d'un nombre de corps en papier correspondant à un conteneur de grande capacité, le nombre comprenant au moins plusieurs milliers de corps en papier, lors du procédé, au moyen d'au moins un contenant (4) réutilisable, dans lequel les corps en papier allongés, en forme de barres sont placés en tant que produit en vrac non trié et non orienté, les corps en papier allongés, en forme de barres étant alimentés vers au moins un dispositif d'alimentation (15) de l'installation de traitement (2), dans un système d'alignement (11) de l'installation de traitement (2), les corps en papier allongés, en forme de barres du nombre de corps en papier correspondant à un conteneur de grande capacité étant alignés au moyen d'au moins un poste d'évacuation par l'intermédiaire d'au moins un système de convoyage (9) de telle sorte que les corps en papier allongés, en forme de barres soient amenés dans une situation de traitement définie, comprenant la position et l'orientation de chaque corps en papier allongé, en forme de barre, de telle sorte que les corps en papier allongés, en forme de barres puissent être alimentés dans la situation de traitement définie vers l'installation de traitement dans laquelle chaque corps en papier allongé, en forme de barre peut être traité exclusivement lorsqu'il se trouve dans la situation de traitement définie et individuellement ou en totalité, des corps en papiers allongés, en forme de barres du nombre de corps en papier correspondant à un conteneur de grande capacité étant alignés dans le système d'alignement (11) de telle sorte que les corps en papier allongés, en forme de barres se trouvent déjà dans la situation de traitement définie lorsqu'ils arrivent vers ou dans l'installation de traitement (2).
